# EUROPEAN PATENT SPECIFICATION

(11) **EP 2 223 116 B1**
(45) Date of publication and mention of the grant of the patent: **19.11.2014**
(21) Application number: 08860118.2
(22) Date of filing: 08.12.2008
(51) Int. Cl.: G01N 33/574

(54) **MARKER PANEL FOR COLORECTAL CANCER**
MARKERPANEL FÜR KOLOREKTALKREBS
PANEL DE MARQUEURS POUR LE CANCER COLORECTAL

(30) Priority: 10.12.2007 EP 07023897; 29.09.2008 EP 08017119
(43) Date of publication of application: 01.09.2010
(73) Proprietor: Roche Diagnostics GmbH, 68305 Mannheim (DE); F. Hoffmann-La Roche AG, 4070 Basel (CH)
(72) Inventor: KARL, Johann, 82380 Peissenberg (DE); ANDRES, Herbert, 82377 Penzberg (DE); GARCZAREK, Ursula, 44309 Dortmund (DE); ROLLINGER, Wolfgang, 86935 Rott (DE); WILD, Norbert, 82538 Geretsried (DE)
(86) International application number: PCT/EP2008/010386
(87) International publication number: WO 2009/074276

(56) References cited:
- EP-A- 0 953 639
- SHIOTA GOSHI ET AL: "Circulating p53 antibody in patients with colorectal cancer: Relation to clinicopathologic features and survival" January 2000 (2000-01), DIGESTIVE DISEASES AND SCIENCES, VOL. 45, NR. 1, PAGE(S) 122-128 , XP002515377 ISSN: 0163-2116 the whole document
- IWASA ET AL: "'Increased expression of seprase, a membrane-type serine protease, is associated with lymph node metastasis in human colorectal cancer'" CANCER LETTERS, NEW YORK, NY, US, vol. 227, no. 2, 28 September 2005 (2005-09-28), pages 229-236, XP005098958 ISSN: 0304-3835
- HENRY LEONARD R ET AL: "Clinical implications of fibroblast activation protein in patients with colon cancer" 15 March 2007 (2007-03-15), CLINICAL CANCER RESEARCH, THE AMERICAN ASSOCIATION FOR CANCER RESEARCH, US, PAGE(S) 1736 - 1741 , XP002474541 ISSN: 1078-0432 the whole document
- GE YUN ET AL: "Fibroblast activation protein (FAP) is upregulated in myelomatous bone and supports myeloma cell survival" 1 April 2006 (2006-04-01), BRITISH JOURNAL OF HAEMATOLOGY, WILEY-BLACKWELL PUBLISHING LTD, GB, PAGE(S) 83 - 92 , XP002474542 ISSN: 0007-1048 the whole document
- SUPPIAH A ET AL: "Anti-p53 autoantibody in colorectal cancer: prognostic significance in long-term follow-up" INTERNATIONAL JOURNAL OF COLORECTAL DISEASE ; CLINICAL AND MOLECULAR GASTROENTEROLOGY AND SURGERY, SPRINGER, BERLIN, DE, vol. 23, no. 6, 11 March 2008 (2008-03-11), pages 595-600, XP019628213 ISSN: 1432-1262

### SEQUENCE LISTING

<110> Roche Diagnostics GmbH F. Hoffmann-La Roche AG
<120> Marker panel for colorectal cancer
<130> 25319 WO
<150> EP 07023897.7
   <151> 2007-12-10
<150> EP 08017119.2
   <151> 2008-09-29
<160> 3
<170> PatentIn version 3.2
<210> 1
   <211> 766
   <212> PRT
   <213> Homo sapiens
<400> 1
   Met Lys Thr Pro Trp Lys Val Leu Leu Gly Leu Leu Gly Ala Ala Ala
   1               5                   10                  15
   Leu Val Thr Ile Ile Thr Val Pro Val Val Leu Leu Asn Lys Gly Thr
               20                  25                  30
   Asp Asp Ala Thr Ala Asp Ser Arg Lys Thr Tyr Thr Leu Thr Asp Tyr
           35                  40                  45
   Leu Lys Asn Thr Tyr Arg Leu Lys Leu Tyr Ser Leu Arg Trp Ile Ser
       50                  55                  60
   Asp His Glu Tyr Leu Tyr Lys Gln Glu Asn Asn Ile Leu Val Phe Asn
   65                  70                  75                  80
   Ala Glu Tyr Gly Asn Ser Ser Val Phe Leu Glu Asn Ser Thr Phe Asp
                   85                  90                  95
   Glu Phe Gly His Ser Ile Asn Asp Tyr Ser Ile Ser Pro Asp Gly Gln
               100                 105                 110
   Phe Ile Leu Leu Glu Tyr Asn Tyr Val Lys Gln Trp Arg His Ser Tyr
           115                 120                 125
   Thr Ala Ser Tyr Asp Ile Tyr Asp Leu Asn Lys Arg Gln Leu Ile Thr
       130                 135                 140
   Glu Glu Arg Ile Pro Asn Asn Thr Gln Trp Val Thr Trp Ser Pro Val
   145 150 155 160
   Gly His Lys Leu Ala Tyr Val Trp Asn Asn Asp Ile Tyr Val Lys Ile
   165 170 175
   Glu Pro Asn Leu Pro Ser Tyr Arg Ile Thr Trp Thr Gly Lys Glu Asp
   180 185 190
   Ile Ile Tyr Asn Gly Ile Thr Asp Trp Val Tyr Glu Glu Glu Val Phe
   195 200 205
   Ser Ala Tyr Ser Ala Leu Trp Trp Ser Pro Asn Gly Thr Phe Leu Ala
   210 215 220
   Tyr Ala Gln Phe Asn Asp Thr Glu Val Pro Leu Ile Glu Tyr Ser Phe
   225 230 235 240
   Tyr Ser Asp Glu Ser Leu Gln Tyr Pro Lys Thr Val Arg Val Pro Tyr
   245 250 255
   Pro Lys Ala Gly Ala Val Asn Pro Thr Val Lys Phe Phe Val Val Asn
   260 265 270
   Thr Asp Ser Leu Ser Ser Val Thr Asn Ala Thr Ser Ile Gln Ile Thr
   275 280 285
   Ala Pro Ala Ser Met Leu Ile Gly Asp His Tyr Leu Cys Asp Val Thr
   290 295 300
   Trp Ala Thr Gln Glu Arg Ile Ser Leu Gln Trp Leu Arg Arg Ile Gln
   305 310 315 320
   Asn Tyr Ser Val Met Asp Ile Cys Asp Tyr Asp Glu Ser Ser Gly Arg
   325 330 335
   Trp Asn Cys Leu Val Ala Arg Gln His Ile Glu Met Ser Thr Thr Gly
   340 345 350
   Trp Val Gly Arg Phe Arg Pro Ser Glu Pro His Phe Thr Leu Asp Gly
   355 360 365
   Asn Ser Phe Tyr Lys Ile Ile Ser Asn Glu Glu Gly Tyr Arg His Ile
   370 375 380
   Cys Tyr Phe Gln Ile Asp Lys Lys Asp Cys Thr Phe Ile Thr Lys Gly
   385 390 395 400
   Thr Trp Glu Val Ile Gly Ile Glu Ala Leu Thr Ser Asp Tyr Leu Tyr
               405                 410                 415
   Tyr Ile Ser Asn Glu Tyr Lys Gly Met Pro Gly Gly Arg Asn Leu Tyr
           420                 425                 430
   Lys Ile Gln Leu Ser Asp Tyr Thr Lys Val Thr Cys Leu Ser Cys Glu
       435                 440                 445
   Leu Asn Pro Glu Arg Cys Gln Tyr Tyr Ser Val Ser Phe Ser Lys Glu
       450                 455                 460
   Ala Lys Tyr Tyr Gln Leu Arg Cys Ser Gly Pro Gly Leu Pro Leu Tyr
   465                 470                 475                 480
   Thr Leu His Ser Ser Val Asn Asp Lys Gly Leu Arg Val Leu Glu Asp
                   485                 490                 495
   Asn Ser Ala Leu Asp Lys Met Leu Gln Asn Val Gln Met Pro Ser Lys
               500                 505                 510
   Lys Leu Asp Phe Ile Ile Leu Asn Glu Thr Lys Phe Trp Tyr Gln Met
           515                 520                 525
   Ile Leu Pro Pro His Phe Asp Lys Ser Lys Lys Tyr Pro Leu Leu Leu
       530                 535                 540
   Asp Val Tyr Ala Gly Pro Cys Ser Gln Lys Ala Asp Thr Val Phe Arg
   545                 550                 555                 560
   Leu Asn Trp Ala Thr Tyr Leu Ala Ser Thr Glu Asn Ile Ile Val Ala
                   565                 570                 575
   Ser Phe Asp Gly Arg Gly Ser Gly Tyr Gln Gly Asp Lys Ile Met His
               580                 585                 590
   Ala Ile Asn Arg Arg Leu Gly Thr Phe Glu Val Glu Asp Gln Ile Glu
           595                 600                 605
   Ala Ala Arg Gln Phe Ser Lys Met Gly Phe Val Asp Asn Lys Arg Ile
       610                 615                 620
   Ala Ile Trp Gly Trp Ser Tyr Gly Gly Tyr Val Thr Ser Met Val Leu
   625                 630                 635                 640
   Gly Ser Gly Ser Gly Val Phe Lys Cys Gly Ile Ala Val Ala Pro Val
                   645                 650                 655
   Ser Arg Trp Glu Tyr Tyr Asp Ser Val Tyr Thr Glu Arg Tyr Met Gly
   660 665 670
   Leu Pro Thr Pro Glu Asp Asn Leu Asp His Tyr Arg Asn Ser Thr Val
   675 680 685
   Met Ser Arg Ala Glu Asn Phe Lys Gln Val Glu Tyr Leu Leu Ile His
   690 695 700
   Gly Thr Ala Asp Asp Asn Val His Phe Gln Gln Ser Ala Gln Ile Ser
   705 710 715 720
   Lys Ala Leu Val Asp Val Gly Val Asp Phe Gln Ala Met Trp Tyr Thr
   725 730 735
   Asp Glu Asp His Gly Ile Ala Ser Ser Thr Ala His Gln His Ile Tyr
   740 745 750
   Thr His Met Ser His Phe Ile Lys Gln Cys Phe Ser Leu Pro
   755 760 765
<210> 2
   <211> 25
   <212> PRT
   <213> Artificial
<220>
   <223> peptide Bi-p53(11-35)
<400> 2
   Glu Pro Pro Leu Ser Gln Glu Thr Phe Ser Asp Leu Trp Lys Leu Leu
   1 5 10 15
   Pro Glu Asn Asn Val Leu Ser Pro Leu
   20 25
<210> 3
   <211> 20
   <212> PRT
   <213> Artificial
<220>
   <223> peptide Bi-p53 (41-60)
<400> 3
   Asp Asp Leu Met Leu Ser Pro Asp Asp Ile Glu Gln Trp Phe Thr Glu
   1 5 10 15
   Asp Pro Gly Pro
   20

## Description

### Detailed Description of the Invention

The present invention relates to a method for assessing colorectal cancer (CRC) in vitro comprising measuring in a sample the concentration and/or activity of a seprase polypeptide and/or fragments thereof and of either anti-p53 and/or osteopontin and/or Ferritin, of optionally one or more other marker of CRC, and using the combined measurement result in the assessment of CRC. Furthermore, it especially relates to a method for assessing CRC from a liquid sample, derived from an individual by measuring seprase and at least one marker selected from the group consisting of anti-p53, Ferritin and osteopontin in said sample. The method according to the present invention can, e.g., be used in the early detection of cancer by screening of asymptomatic individuals or in the surveillance of patients who undergo surgery.

Cancer remains a major public health challenge despite progress in detection and therapy. Cancer cells are characterized by the production of cancer-associated marker proteins. Cancer-associated proteins are found both in the tissues and in the bodily fluids of an individual who carries cancer cells. Their levels usually are low at the early stages of the carcinogenic progress and increase during the disease's progression and only in rare cases proteins are observed showing a decreased level in the course of disease progression. The sensitive detection of these proteins is an advantageous and promising approach for the diagnosis of cancer, in particular in an early stage diagnosis of cancer. The most prevalent cancer types are breast cancer (BC), lung cancer (LC) and colorectal cancer (CRC).

The most important therapeutic approaches for solid tumors are:
a) surgical resection of the tumor,
b) chemotherapy,
c) treatment with biologicals, like anti-tumor antibodies or anti-angiogenic antibodies and
d) a combination of the above methods.

Surgical resection of the tumors is widely accepted as a first line treatment for early stage solid tumors. Most cancers, however, are detected only when they become symptomatic, i.e. when patients already are in a rather late stage of disease progression.

Colorectal cancer most frequently progresses from adenomas (polyps) to malignant carcinomas.

The staging of cancer is the classification of the disease in terms of extent, progression, and severity. It groups cancer patients so that generalizations can be made about prognosis and the choice of therapy.

The different stages of CRC used to be classified according to Dukes' stages A to D. Today, the TNM system is the most widely used classification of the anatomical extent of cancer. It represents an internationally accepted, uniform staging system. There are three basic variables: T (the extent of the primary tumor), N (the status of regional lymph nodes) and M (the presence or absence of distant metastases). The TNM criteria are published by the UICC (International Union Against Cancer), Sobin, L.H., Wittekind, Ch. (eds): TNM Classification of Malignant Tumours, sixth edition, 2002). Once the TNM status is determined the patients are grouped into disease stages that are denoted by Roman numerals ranging form I to IV with IV being the most advanced disease stage. TNM staging and UICC disease stages correspond to each other as shown in the following Table taken from Sobin L.H. and Wittekind (eds.) *supra.*

### Interrelation of TNM staging and UICC disease stages:

| UICC disease stage | T staging | N staging | M staging |
|---|---|---|---|
| Stage 0 | Tis | N0 | M0 |
| Stage I | T1, T2 | N0 | M0 |
| Stage IIA | T3 | N0 | M0 |
| Stage IIB | T4 | N0 | M0 |
| Stage IIIA | T1, T2 | N1 | M0 |
| Stage IIIB | T3, T4 | N1 | M0 |
| Stage IIIC | AnyT | N2 | M0 |
| Stage IV | AnyT | AnyN | M1 |

What is especially important is, that early diagnosis of CRC translates to a much better prognosis. Malignant tumors of the colorectum arise from benign tumors, i.e. from adenoma. Therefore, best prognosis have those patients diagnosed at the adenoma stage. Patients diagnosed as early as in stage Tᵢₛ, N0, M0 or T1-3; N0; M0, if treated properly have a more than 90% chance of survival 5 years after diagnosis as compared to a 5-years survival rate of only 10% for patients diagnosed when distant metastases are already present.

Current detection methods including imaging methods, such as x-ray or nuclear resonance imaging in theory might at least partially be appropriate for use as a general screening tool. However, they are very costly and not affordable to health care systems for a general and broad use in mass screenings of large numbers of subjects, particularly for subjects without any tumor symptoms.

Thus, it is an object of the present invention to provide a simple and cost-efficient procedure of tumor assessments, e.g. to identify individuals suspect of having colorectal cancer. For this purpose, a tumor marker which is detectable in body fluids, e.g. blood or serum or plasma or a panel of such markers, would be desirable.

Despite the large and ever growing list of candidate protein markers in the field of CRC, to date clinical/diagnostic utility of these molecules is not known or established. It would appear that currently no method based on a bodily fluid as a sample is available that would be appropriate in screening for CRC.

Diagnostic blood tests based on the detection of carcinoembryonic antigen (CEA), a tumor-associated glycoprotein, are available. CEA is increased in 95% of tissue samples obtained from patients with colorectal, gastric, and pancreatic cancers and in the majority of breast, lung, and head and neck carcinomas (Goldenberg, D.M. et al., J. Natl. Cancer Inst. (Bethesda) 57 (1976) 11-22). Elevated CEA levels have also been reported in patients with nonmalignant disease, and many patients with colorectal cancer have normal CEA levels in the serum, especially during the early stage of the disease (Carriquiry, L.A. and Pineyro, A., Dis. Colon Rectum 42 (1999) 921-929; Herrera, M.A. et al., Ann. Surg. 183 (1976) 5-9; Wanebo, H.J. et al., N. Engl. J. Med. 299 (1978) 448-451). The utility of CEA as measured from serum or plasma in detecting recurrences is reportedly controversial and has yet to be widely applied (Martell, R.E. et al., Int. J. Biol. Markers 13 (1998) 145-149; Moertel, C.G. et al., JAMA 270 (1993) 943-947).

In light of the available data, serum CEA determination possesses neither the sensitivity nor the specificity to enable its use as a screening test for colorectal cancer in the asymptomatic population (Reynoso, G. et al., JAMA 220 (1972) 361-365; Sturgeon, C., Clin. Chem. 48 (2002) 1151-1159).

Currently all screening methods for CRC rely on the use of stool as a sample. Samples taken from stool have the implicit advantages that their sampling is easily possible by non-invasive means and that this sample has some tissue specificity, i.e. for intestinal tissue. Screening for CRC from a bodily fluid sample to the contrary is considered almost impossible, because the gut lumen may be vied as being on the outside (ectoderm) of the human body, having a poor blood supply. This in turn would mean additional hurdles to a screening for CRC from a bodily fluid sample like whole blood serum or plasma.

The guaiac test is currently most widely used as a screening assay for CRC from stool. The guaiac test, however, has both poor sensitivity as well as poor specificity. The sensitivity of the guaiac-based fecal occult blood tests is ∼26%, which means 74% of patients with malignant lesions will remain undetected (Ahlquist, D.A., Gastroenterol. Clin. North Am. 26 (1997) 41-55).

The visualization of precancerous and cancerous lesions represents the best approach to early detection, but colonoscopy is invasive with significant costs, risks, and complications (Silvis, S.E. et al., JAMA 235 (1976) 928-930; Geenen, J.E. et al., Am. J. Dig. Dis. 20 (1975) 231-235; Anderson, W.F. et al., J. Natl. Cancer Institute 94 (2002) 1126-1133).

The sensitivity and specificity of diagnostic alternatives to the guaiac test have been recently investigated by Sieg, A. et al., Int. J. Colorectal Dis. 14 (1999) 267-271. Especially the measurements of hemoglobin and of the hemoglobin-haptoglobin complex, respectively, from a stool specimen have been compared. It has been noted that the hemoglobin assay has an unsatisfactory sensitivity for the detection of colorectal neoplasms. Whereas cancer in its progressed carcinoma stage is detected with a sensitivity of about 87% the earlier tumor stages are not detected with a sufficient sensitivity. The hemoglobin-haptoglobin complex assay was more sensitive in the detection of earlier stages of CRC. This more sensitive detection was accompanied by a poor specificity. Since poor specificity, however, translates to a high number of unnecessary secondary investigations, like colonoscopy, an assay with a poor specificity also does not meet the requirements of a generally accepted screening assay.

Calprotectin has been described as an alternative biomarker for the detection of CRC from stool samples in US 5,455,160 and correspondingly in the scientific literature by Roseth, A.G., et al. (Scand. J. Gastroenterol. 27 (1992) 793-798; Scand. J. Gastroenterol. 28 (1993) 1073-1076). Although calprotectin is a marker of inflammatory diseases its potential as a marker for the detection of CRC from stool is documented by several publications (Johne, B., et al., Scand. J. Gastroenterol. 36 (2001) 291-296; Limburg, P.J., et al., Am. J. Gastroenterol. 98 (2003) 2299-2305; Hoff, G., et al., Gut 53 (2004) 1329-1333). While the sensitivity and specificity of calprotectin are comparable to the immunological hemoglobin assay, calprotectin appears to have some characteristics favorable for a diagnostic biomarker as compared to hemoglobin. It is evenly distributed in feces, it is stable at room temperature making mail delivery of the sample to the laboratory feasible and it shows no interference with food components or pharmaceutical compounds (Ton, H., et al., Clin. Chim. Acta 292 (2000) 41-54). However, elevated concentrations of calprotectin, the heterodimer of S100A8 and S100A9, were detected in stool samples from patients suffering from CRC, Crohn's disease or inflammatory bowel disease. These results are in agreement with the more general role of calprotectin in inflammation (Ryckman, C., et al., J. Immunol. 170 (2003) 3233-3242). Hence, the use of calprotectin in gastroenterology is not limited to the detection of CRC but extends to other disesases, especially inflammatory bowel disease as reviewed by Poullis, A., et al. (J. Gastroenterol. Hepatol. 18 (2003) 756-762).

A further alternative method to the guaiac test for detection of CRC in stool has been published recently and consists in the detection of the colorectal cancer-specific antigen, "minichromosome maintenance protein 2" (MCM2) by immunohistochemistry in colonic cells shed into stool. Due to the small study size, conclusion on the diagnostic value for detection of colorectal cancer is preliminary. However, the test seems to have only limited sensitivity to detect right-sided colon cancer (Davies, R.J. et al., Lancet 359 (2002) 1917-1919).

Recently, an assay for detection of pyruvate kinase M2 isoenzyme (M2-PK) has been introduced into the market (Schebo Biotech, Gießen, Germany). A comparison of the guaiac assay to the immuno assays for hemoglobin and M2-PK has for example been performed by Vogel, T. et. al., Dtsch. Med. Wochenschr. 130 (2005) 872-877. They show that the immunological assays are superior to the guaiac test and that at comparable specificity the M2-PK assay is less sensitive in detecting CRC as compared to the hemoglobin assay. Yet, the authors conclude that the usefulness of both these stool based assays is still questionable.

The identification of an early CRC tumor marker or marker panel that would allow for reliable cancer detection or provide for early prognostic information by non-invasive means from a bodily fluid sample would greatly aid in the diagnosis and in the screening for this disease. Quite a number of patients and of providers of diagnostic services prefers a bodily fluid like whole blood, serum or plasma over the handling of a stool sample.

It is especially important to improve the early diagnosis of CRC, since for patients diagnosed early on chances of survival are much higher as compared to those diagnosed at a progressed stage of disease. Therefore, an urgent clinical need exists to improve the diagnosis of CRC, especially from a bodily fluid sample.

It was the task of the present invention to investigate whether a new marker or marker panel can be identified which may aid in assessing colorectal cancer (CRC) in vitro. Surprisingly, it has been found that the use of a marker panel comprising at least the measurement results for seprase and for anti-p53, or for osteopontin and/or for Ferritin is very advantageous in the in vitro assessment of CRC from a bodily fluid sample.

In order to be of clinical utility, a new diagnostic marker as a single marker should be comparable to other markers known in the art, or better. Or, a new marker should lead to a progress in diagnostic sensitivity and/or specificity either if used alone or in combination with one or more other markers, respectively. The diagnostic sensitivity and/or specificity of a test is best assessed by its receiver-operating characteristics, which will be described in detail below. As will be shown in detail below the new marker panels of the present invention have surprisingly been found that significantly improve the diagnostic accuracy, especially in the screening for CRC.

In a preferred embodiment the present invention relates to a method for assessing colorectal cancer (CRC) in vitro comprising measuring in a sample the concentration and/or activity of a seprase polypeptide and/or fragments thereof, of anti-p53, osteopontin and/or Ferritin, of optionally one or more other marker of CRC, and using the combined measurement result for seprase, anti-p53, osteopontin and/or Ferritin and optionally of the one or more other marker in the assessment of CRC.

Surprisingly, it was found that a decreased concentration and/or activity of a seprase polypeptide and/or fragments thereof in the test sample is associated with the risk and/or occurrence of cancer, especially of CRC. The marker seprase can be combined with the marker anti-p53, osteopontin and/or Ferritin and, if required, with other markers for CRC, like CEA and/or CYFRA21-1.

One embodiment of the present invention refers to the mass screening of a population to distinguish between individuals that are probably free from colorectal cancer and individuals which might be classified as "suspect" cases, i.e. individuals, which might have CRC. The latter group of individuals could then be subjected to further diagnostic procedures, e.g. by imaging methods or other suitable means.

A further embodiment of the present invention refers to an improvement of tumor marker panels which are suitable for the assessment of colorectal cancer.

In one embodiment the present invention is directed to a method for assessing CRC in vitro by biochemical marker, comprising measuring in a sample the concentration and/or activity of seprase and using the measurement results, particularly the concentration or activity determined in the assessment of colorectal cancer.

The present invention is also directed to a method for assessing CRC in vitro by biochemical marker, comprising measuring in a sample the concentration and/or activity of seprase, of a marker selected from the group consisting of anti-p53, osteopontin and Ferritin and of one or more other markers, and using the measurement results, particularly concentrations, determined in the assessment of colorectal cancer. Preferred markers for use in combination with seprase, anti-p53, osteopontin and/or Ferritin are markers which are general tumor markers (i.e. markers which are not specific for a single tumor type) or, on the other hand, specific tumor markers (markers which are specific for a single tumor type). Preferred the one or more other marker is Carcinoembryonic antigen (CEA) and/or CYFRA21-1. Preferably, these markers are used in any combination together with seprase.

A cut-off value for an individual marker or a marker combination can for example be determined from the testing of a group of healthy individuals. Preferably the cut-off is set to result in a specificity of 90%, also preferred the cut-off is set to result in a specificity of 95%, or also preferred the cut-off is set to result in a specificity of 98%. A value for an individual marker or a marker combination above the cut-off value can for example be indicative for the presence of cancer. As the skilled artisan appreciates, for certain markers, like for seprase, the same is true for a value below a cut-off value.

The present invention, in a preferred embodiment, relates to a method for assessing CRC, comprising measuring seprase and anti-p53 and using the combined measurement values for assessment of CRC.

The present invention, in a preferred embodiment, relates to a method for assessing CRC, comprising measuring seprase and osteopontin and using the combined measurement values for assessment of CRC.

The present invention, in a preferred embodiment, relates to a method for assessing CRC, comprising measuring seprase and Ferritin and using the combined measurement values for assessment of CRC.

The present invention, in a further preferred embodiment, relates to a method for assessing CRC, comprising measuring seprase, Ferritin, CEA and osteopontin and using the combined measurement values for assessment of CRC.

The present invention, in a further preferred embodiment, relates to a method for assessing CRC, comprising measuring seprase, Ferritin, CEA, osteopontin and anti-p53 and using the combined measurement values for assessment of CRC.

The present invention, in a further preferred embodiment, relates to a method for assessing CRC, comprising measuring seprase, Ferritin, CEA, CYFRA21-1, osteopontin and anti-p53 and using the combined measurement values for assessment of CRC.

The present invention, in a preferred embodiment, also relates to the use of a marker panel comprising at least seprase and anti-p53 in the assessment of colorectal cancer.

The present invention, in a preferred embodiment, also relates to the use of a marker panel comprising at least seprase and anti-p53 in the assessment of colorectal cancer.

The present invention, in a preferred embodiment, also relates to the use of a marker panel comprising at least seprase and osteopontin in the assessment of colorectal cancer.

The present invention, in a preferred embodiment, also relates to the use of a marker panel comprising at least seprase and Ferritin in the assessment of colorectal cancer.

The present invention, in a preferred embodiment, also relates to the use of a marker panel comprising at least seprase and CEA in the assessment of colorectal cancer.

The present invention, in a preferred embodiment, also relates to the use of a marker panel comprising at least seprase and CYFRA21-1 in the assessment of colorectal cancer.

The present invention also relates to the use of a seprase polypeptide and/or fragments thereof in the assessment of colon cancer, wherein a decreased concentration and/or activity of seprase and/or fragments thereof is indicative for cancer.

The present invention also relates to the use of an antibody directed against a seprase polypeptide and/or fragments thereof in the assessment of colon cancer, wherein a decreased concentration and/or activity of seprase and/or fragments thereof is indicative for colon cancer.

The present invention also relates to the use of a reagent for measuring the enzymatic activity of a seprase polypeptide and/or fragments thereof in the assessment of colon cancer, wherein a decreased concentration and/or activity of seprase and/or of enzymatically active fragments thereof is indicative for colon cancer.

The present invention also relates to a device comprising the means for performing the method according to the present invention or for making use of the marker combinations disclosed.

The present invention also provides for a kit comprising the means for performing the method according to the present invention, i.e. the means for at least measuring seprase and a marker selected from the group consisting of anti-p53, osteopontin and Ferritin.

The present invention also provides a kit for performing the method according to the present invention comprising at least the reagents required to specifically measure seprase and anti-p53, respectively, and optionally auxiliary reagents for performing the measurement.

The present invention also provides a kit for performing the method according to the present invention comprising at least the reagents required to specifically measure seprase and osteopontin, respectively, and optionally auxiliary reagents for performing the measurement.

The present invention also provides a kit for performing the method according to the present invention comprising at least the reagents required to specifically measure seprase and Ferritin, respectively, and optionally auxiliary reagents for performing the measurement.

The present invention also provides a kit for performing the method according to the present invention comprising at least the reagents required to specifically measure seprase and CYFRA21-1, respectively, and optionally auxiliary reagents for performing the measurement.

The present invention also provides a kit for performing the method according to the present invention comprising at least the reagents required to specifically measure seprase and CEA, respectively, and optionally auxiliary reagents for performing the measurement.

In one preferred embodiment the present invention relates to a method for assessing colorectal cancer in vitro comprising measuring in a sample the concentration and/or activity of (a) a seprase polypeptide and/or fragments thereof, (b) optionally one or more other marker of CRC, and (c) using the measurement result of step (a) and optionally of step (b) in the assessment of CRC.

In a preferred embodiment the present invention relates to a method for assessing colorectal cancer (CRC) in vitro comprising measuring in a sample the concentration and/or activity of (a) a seprase polypeptide and/or fragments thereof, (b) anti-p53, osteopontin and/or Ferritin, (c) optionally one or more other marker of CRC, and (d) using the combined measurement result of step (a) and (b) and optionally of step (c) in the assessment of CRC.

In preferred embodiments the concentration and/or activity of seprase is combined with the measurement for at least anti-p53, osteopontin or Ferritin and the combined value is correlated to an underlying diagnostic question like e.g. likelihood of presence or absence of CRC, stage of disease, disease progression, or response to therapy.

Human seprase (alias fibroblast activation protein (FAP)) is a 170 kDa membrane-bound glycoprotein having gelatinase and dipeptidyl peptidase activity. It consists of two identical monomeric units (Uni Prot KB database Accession No. P 27487) characterized by the sequence given in SEQ ID NO:1. The seprase monomer comprises 766 amino acids and has an apparent molecular weight of 97 kDa (SDS-PAGE) (Pineiro-Sanchez, et al., J. Biol. Chem. 272 (1997) 7595-7601; Park, et al., J. Biol. Chem. 274 (1999) 36505-36512). A soluble form of seprase is the antiplasmin-cleaving enzyme APCE (Lee, et al., Blood 103 (2004) 3783-3788; Lee, et al., Blood 107 (2006) 1397-1404). The N-terminal amino acid sequence of APCE corresponds to residues 24-38 of seprase which is predicted to have its first 6 N-terminal residues within the fibroblast cytoplasm, followed by a 20-residue transmembrane domain and then a 734 residue extracellular C-terminal catalytic domain (Goldstein, et al., Biochim Biophys Acta. 1361 (1997) 11-19; Scanlan, et al., Proc Natl Acad Sci USA 91 (1994) 5657-5661).

Piñeiro-Sánchez et al. *(supra)* found that an increased expression of seprase correlates with the invasive phenotype of human melanoma and carcinoma cells. Henry et al., Clin. Cancer Res. 13 (2007) 1736-1741, describe that human colon tumor patients having high levels of stromal seprase are more likely to have aggressive disease progression and potential development of metastases or recurrence.

Iwasa et al., 2005 discloses that the increased expression of seprase activity is associated with lymph node metastasis in human colorectal cancer.

Hence, none of the above documents of the art suggests that a decreased concentration and/or activity of a seprase polypeptide and/or fragments thereof in body fluids would be indicative for cancer.

Surprisingly, it was found in the present invention that a determination of the concentration and/or the activity, e.g. the enzymatic activity of a seprase polypeptide and/or fragments thereof, particularly of APCE in a body fluid, allows the assessment of cancer disease, e.g. of lung, breast, colon, or kidney cancer disease, and in particular of lung cancer disease. Even more surprisingly, it was found that a decreased concentration and/or activity of seprase or fragments thereof in a sample compared to normal controls is indicative for the risk or occurrence of cancer.

As used herein, each of the following terms has the meaning associated with it in this section.

The articles "a" and "an" are used herein to refer to one or to more than one (i.e. to at least one) of the grammatical object of the article. By way of example, "a marker" means one marker or more than one marker. The term "at least" is used to indicate that optionally one or more further objects may be present. By way of example, a marker panel comprising at least (the markers) seprase and CYFRA21-1 may optionally comprise one or more other marker.

The expression "one or more" denotes 1 to 50, preferably 1 to 20 also preferred 2, 3, 4, 5, 6, 7, 8, 9, 10, 12, or 15.

The term "marker" or "biochemical marker" as used herein refers to a molecule to be used as a target for analyzing a patient's test sample. Examples of such molecular targets are proteins or polypeptides. Proteins or polypeptides used as a marker in the present invention are contemplated to include naturally occurring variants of said protein as well as fragments of said protein or said variant, in particular, immunologically detectable fragments. Immunologically detectable fragments preferably comprise at least 6, 7, 8, 10, 12, 15 or 20 contiguous amino acids of said marker polypeptide. One of skill in the art would recognize that proteins which are released by cells or present in the extracellular matrix may be damaged, e.g., during inflammation, and could become degraded or cleaved into such fragments. Certain markers are synthesized in an inactive form, which may be subsequently activated by proteolysis. As the skilled artisan will appreciate, proteins or fragments thereof may also be present as part of a complex. Such complex also may be used as a marker in the sense of the present invention. Variants of a marker polypeptide are encoded by the same gene, but may differ in their isoelectric point (=PI) or molecular weight (=MW), or both e.g., as a result of alternative mRNA or premRNA processing. The amino acid sequence of a variant is to 95% or more identical to the corresponding marker sequence. In addition, or in the alternative a marker polypeptide or a variant thereof may carry a post-translational modification. Non-limiting examples of posttranslational modifications are glycosylation, acylation, and/or phosphorylation.

A "marker of cancer" and in particular a "marker of colon cancer or colorectal cancer" in the sense of the present invention is any marker that if combined with the marker combination comprising seprase and a marker selected from the group consisting of anti-p53, osteopontin and Ferritin adds relevant information in the assessment of CRC. The information is considered relevant or of additive value if at a given specificity the sensitivity, or if at a given sensitivity the specificity, respectively, for the assessment of CRC can be improved by including said marker into a marker combination already comprising the marker seprase and a marker selected from the group consisting of anti-p53, osteopontin and Ferritin. In the preferred embodiment of CRC assessment, the improvement in sensitivity or specificity, respectively, is statistically significant at a level of significance of p = .05, .02, .01 or lower.

The term "sample" as used herein refers to a biological sample obtained for the purpose of evaluation in vitro. In the methods of the present invention, the sample or patient sample preferably comprises any body fluid. Preferred samples are whole blood, serum, plasma with plasma or serum being most preferred.

The term "assessing cancer" and in particular "assessing colon cancer or colorectal cancer" is used to indicate that the method according to the present invention will (alone or together with other markers or variables, e.g., the criteria set forth by the UICC (see above)) e.g., aid the physician to establish or confirm the absence or presence of CRC or aid the physician in the prognosis, the detection of recurrence (follow-up of patients after surgery) and/or the monitoring of treatment, especially of chemotherapy.

As the skilled artisan will appreciate, any such assessment is made in vitro. The patient sample is discarded afterwards. The patient sample is solely used for the in vitro diagnostic method of the invention and the material of the patient sample is not transferred back into the patient's body. Typically, the sample is a liquid sample, e.g., whole blood, serum, or plasma.

The term "measurement" preferably comprises a qualitative, semi-quantitative or a quantitative measurement of a marker in a sample. In a preferred embodiment the measurements in a method according to the present invention are quantitative measurements.

### Seprase:

According to the present invention, the term "seprase polypeptide and/or fragments thereof" refers to monomeric or multimeric, particularly dimeric polypeptides. Further, the term "seprase polypeptide and/or fragments thereof" particularly refers to soluble forms of seprase and/or fragments thereof, particularly antiplasmin-cleaving enzyme (APCE) as well as to seprase and/or seprase fragments present in form of a complex with another polypeptide.

Seprase polypeptides, particularly soluble forms of seprase polypeptides and/or fragments thereof, are preferably detected in appropriate samples, particularly in body fluids. Preferred samples are body fluids, such as blood, plasma, serum, sputum and urine. Preferably, the sample is derived from a human subject, e.g. a tumor patient or a person in risk of a tumor or a person suspected of having a tumor.

According to the present invention, the concentration and/or activity of a seprase polypeptide and/or fragments thereof is determined. In one embodiment, the marker seprase is specifically measured from a sample by use of a specific binding agent.

Various immunodiagnostic procedures may be used to reach a result comparable to the achievements of the present invention. For example, alternative strategies to generate antibodies may be used. Such strategies comprise amongst others the use of synthetic peptides, representing an epitope of seprase for immunization. Alternatively, DNA immunization also known as DNA vaccination may be used.

Preferably, seprase is detected in a sandwich-type assay format. In such assay, a first specific binding agent is used to capture seprase on the one side and a second specific binding agent, which is labeled to be directly or indirectly detectable, is used on the other side. The specific binding agents used in a sandwich-type assay format may be antibodies specifically directed against seprase. On the one hand, the detection may be carried out by using different capturing and labeled antibodies, i.e. antibodies which recognize different epitopes on the seprase polypeptide. On the other hand, a sandwich-type assay may also be carried out with a capture and labeling antibody which is directed against the same epitope of seprase. In this embodiment, only di- and multimeric forms of seprase may be detected.

According to a further embodiment of the invention, the marker seprase is specifically measured from a sample by use of a test regimen suitable for detecting the enzymatic activity of seprase. The enzymatic activity may e.g. be a gelatinase activity or a peptidase activity. Gelatinase activity may e.g. be determined by zymography using gelatine zymograms. Peptidase activity may e.g. be determined by fluorescent assays using appropriate labeled peptide substrates. Assays for the measurement of seprase activity are e.g. described by Lee et al. (2006), *supra.*

### Osteopontin (OPN):

OPN is found in normal plasma, urine, milk and bile (US 6,414,219; US 5,695,761; Denhardt, D.T. and Guo, X., FASEB J. 7 (1993) 1475-1482; Oldberg, A., et al., PNAS 83 (1986) 8819-8823; Oldberg, A., et al., J. Biol. Chem. 263 (1988) 19433-19436; Giachelli, C.M., et al., Trends Cardiovasc. Med. 5 (1995) 88-95). The human OPN protein and cDNA have been isolated and sequenced (Kiefer M.C., et al., Nucl. Acids Res. 17 (1989) 3306).

OPN functions in cell adhesion, chemotaxis, macrophage-directed interleukin-10 (IL-10) suppression, stress-dependent angiogenesis, prevention of apoptosis, and anchorage-independent growth of tumor cells by regulating cell-matrix interactions and cellular signaling through binding with integrin and CD44 receptors. While constitutive expression of OPN exists in several cell types, induced expression has been detected in T-lymphocytes, epidermal cells, bone cells, macrophages, and tumor cells in remodeling processes such as inflammation, ischemia-reperfusion, bone resorption, and tumor progression (reviewed by Wai, P.Y. and Kuo, P.C., J. Surg. Res. 121 (2004) 228-241).

OPN is known to interact with a number of integrin receptors. Increased OPN expression has been reported in a number of human cancers, and its cognate receptors (av-b3, av-b5, and av-b1 integrins and CD44) have been identified. In vitro studies by Irby, R.B., et al., Clin. Exp. Metastasis 21 (2004) 515-523 indicate that both endogenous OPN expression (via stable transfection) as well as exogenous OPN (added to culture medium) enhanced the motility and invasive capacity of human colon cancer cells in vitro. OPN appeared to regulate motility though interaction with CD44. OPN expression also reduced intercellular (homotypic) adhesion, which is regarded as a characteristic of metastatic cancer cells. Stable transfection of four poorly tumorigenic human colon cancer cell lines with OPN also resulted in enhanced tumorigenicity in vivo with increased proliferation and increased CD31 positive micro vessel counts, concordant with the degree of OPN expression. Fragments of OPN may also be used in a method according to the present invention.

Mor, G., et al., Proc. Natl. Acad. Sci. USA 102 (2005) 7677-7682 report a blood (serum) test for the early diagnosis of epithelial ovarian cancer based on the simultaneous quantization of OPN and three other analytes.

In a preferred embodiment the present invention relates to a method for assessing CRC in vitro by biochemical markers, comprising measuring in a sample the concentration of osteopontin and of seprase, combining the measured values and using the combined value in the assessment of CRC.

### Carcinoembryonic antigen (CEA):

CEA (carcinoembryonic antigen) is a monomeric glycoprotein (molecular weight approx. 180.000 Dalton) with a variable carbohydrate component of approx. 45-60% (Gold, P. and Freedman, S.O., J. Exp Med 121 (1965) 439-462). CEA, like AFP, belongs to the group of carcinofetal antigens that are produced during the embryonic and fetal period. The CEA gene family consists of about 17 active genes in two subgroups. The first group contains CEA and the Non-specific Cross-reacting Antigens (NCA); the second group contains the Pregnancy-Specific Glycoproteins (PSG).

CEA is mainly found in the fetal gastrointestinal tract and in fetal serum. It also occurs in slight quantities in intestinal, pancreatic, and hepatic tissue of healthy adults. The formation of CEA is repressed after birth, and accordingly serum CEA values are hardly measurable in healthy adults.

High CEA concentrations are frequently found in cases of colorectal adenocarcinoma (Fateh-Modhadam, A. et al. (eds.), Tumormarker und ihr sinnvoller Einsatz, Juergen Hartmann Verlag GmbH, Marloffstein-Rathsberg (1993), ISBN-3-926725-07-9). Slight to moderate CEA elevations (rarely > 10 ng/mL) occur in 20-50 % of benign diseases of the intestine, the pancreas, the liver, and the lungs (e.g. liver cirrhosis, chronic hepatitis, pancreatitis, ulcerative colitis, Crohn's Disease, emphysema (Fateh-Moghadam, A., et al., supra). Smokers also have elevated CEA values.

The main indication for CEA determinations is therapy management and the follow-up of patients with colorectal carcinoma. CEA determinations are not recommended for cancer-screening in the general population. CEA concentrations within the normal range do not exclude the possible presence of a malignant disease.

The antibodies in the assay manufactured by Roche Diagnostics react with CEA and (as with almost all CEA detection methods) with the meconium antigen (NCA2). Cross-reactivity with NCA1 is 0.7 % (Hammarstrom, S., et al., Cancer Res. 49 (1989) 4852-4858; and Bormer, O.P., Tumor Biol. 12 (1991) 9-15). CEA has been measured on an Elecsys^{®} analyzer using Roche product number 11731629 according to the manufacturer's instructions.

### CYFRA21-1:

An assay for "CYFRA21-1" specifically measures a soluble fragment of cytokeratin 19 as present in the circulation. The measurement of CYFRA21-1 is typically based upon two monoclonal antibodies (Bodenmueller, H., et al., Int. J. Biol. Markers 9 (1994) 75-81). In the CYFRA21-1 assay from Roche Diagnostics, Germany, the two specific monoclonal antibodies (KS 19.1 and BM 19.21) are used and a soluble fragment of cytokeratin 19 having a molecular weight of approx. 30,000 Daltons is measured. Cytokeratins are structural proteins forming the subunits of epithelial intermediary filaments. Twenty different cytokeratin polypeptides have so far been identified. Due to their specific distribution patterns they are eminently suitable for use as differentiation markers in tumor pathology. Intact cytokeratin polypeptides are poorly soluble, but soluble fragments can be detected in serum (Bodenmueller, H., et al., supra).

CYFRA21-1 is a well-established marker for Non-Small-Cell Lung Carcinoma (NSCLC). The main indication for CYFRA21-1 is monitoring the course of non-small cell lung cancer (NSCLC) (Sturgeon, C., Clinical Chemistry 48 (2002) 1151-1159).

In primary diagnosis high CYFRA21-1 serum levels indicate an advanced tumor stage and a poor prognosis in patients with non-small-cell lung cancer (van der Gaast, A.., et al., Br. J. Cancer 69 (1994) 525-528). A normal or only slightly elevated value does not rule out the presence of a tumor. Successful therapy is documented by a rapid fall in the CYFRA21-1 serum level into the normal range. A constant CYFRA21-1 value or a slight or only slow decrease in the CYFRA21-1 value indicates incomplete removal of a tumor or the presence of multiple tumors with corresponding therapeutic and prognostic consequences. Progression of the disease is often shown earlier by increasing CYFRA21-1 values than by clinical symptomatology and imaging procedures.

Slightly elevated values (up to 10 ng/mL) are rarely found in marked benign liver diseases and renal failure. There is no correlation with sex, age or smoking. The values for CYFRA21-1 are also unaffected by pregnancy.

CYFRA21-1 preferably is measured on an Elecsys^{®} analyzer using Roche product number 11820966 according to the manufacturer's instructions.

### Ferritin:

Ferritin is a macromolecule with a molecular weight of at least 440 kD (depending on the iron content) and consists of a protein shell (apoferritin) of 24 subunits and an iron core containing an average of approx. 2500 Fe³⁺ ions (in liver and spleen ferritin) (Wick, M. et al., Ferritin in Iron Metabolism - Diagnosis of Anemieas (second edition). Springer-Verlag 1995;ISBN 3-211-82525-8 and ISBN 0-387-82525-8) Ferritin tends to form oligomers, and when it is present in excess in the cells of the storage organs there is a tendency for condensation to semicrystalline hemosiderin to occur in the lysosomes.

At least 20 isoferritins can be distinguished with the aid of isoelectric focusing (Arosio, P. et al., Heterogeneity of ferritin II: Immunological aspects. In: Albertini A, Arosio P, Chiancone E, Drysdale J (eds). Ferritins and isoferritins as biochemical markers. Elsevier, Amsterdam 1984;33-47). This microheterogeneity is due to differences in the contents of the acidic H and weakly basic L subunits. The basic isoferritins are responsible for the long-term iron storage function, and are found mainly in the liver, spleen, and bone marrow (Kaltwasser, J.P. et al., Serumferritin: Methodische und Klinische Aspekte. Springer Verlag (1980)). Acidic isoferritins are found mainly in the myocardium, placenta, and tumor tissue. They have a lower iron content and presumably function as intermediaries for the transfer of iron in various syntheses (Morikawa, K. et al., Leuk. Lymphoma 18(5-6) (1995) 429-433; Borch-Iohnson, B., Analyst 120(3) (1995) 891-903; Cook, J. et al., Adv. Exp. Med. Biol. 356 (1994) 219-228).

The determination of ferritin is a suitable method for ascertaining the iron metabolism situation. Determination of ferritin at the beginning of therapy provides a representative measure of the body's iron reserves. Clinically, a threshold value of 20 µg/L (ng/mL) has proved useful in the detection of prelatent iron deficiency. This value provides a reliable indication of exhaustion of the iron reserves that can be mobilized for hemoglobin synthesis. When the ferritin level is elevated and the possibility of a distribution disorder can be ruled out, this is a manifestation of iron overloading in the body. 400 µg/L (ng/mL) ferritin is used as the threshold value. Elevated ferritin values are also encountered with the following tumors: acute leukemia, Hodgkin's disease and carcinoma of the lung, liver and prostate. The determination of ferritin has proved to be of value in liver metastasis. Studies indicate that 76% of all patients with liver metastasis have ferritin values above 400 µg/L (ng/mL). Reasons for the elevated values could be cell necrosis, blocked erythropoiesis or increased synthesis in tumor tissue.

On the other hand it was described as characteristic, that serum Ferritin levels did not increase in gastrointestinal cancer (Niitsu, Y. et al., Rinsho Ketsueki 21 (1980) 1135-1143). Furthermore, mean serum ferritin levels have been reported to be significantly lower in patients with advanced colorectal cancer than in controls (Kishida, T. et al., J. Gastroenterol. 29 (1994) 19-23). The decrease of serum Ferritin levels is caused by continous bleeding and this blood loss is related to the size and site of the tumor (Feng, L. et al., J. Gastroenterol. 34 (1999) 195-199).

Ferritin preferably is measured on an Elecsys^{®} analyzer using Roche product number 11820982 according to the manufacturer's instructions.

### Anti-p53:

25 years ago p53 antibodies were discovered during the course of tumor-associated antigen screening in breast cancer patients without the knowledge of mutations of the p53 gene (Crawford, L. et al., Int. J. Cancer 30 (1982) 403-408). The tumor suppressor p53 is a phosphoprotein barely detectable in the nucleus of normal cells (Benchimol, S. et al., EMBO J. 1 (1982) 1055-1062). On cellular stress, particularly that induced by DNA damage, p53 can arrest cell cycle progression, thus allowing the DNA to be repaired or it can lead to apoptosis. In cancer cells that bear a mutant p53, this protein is no longer able to control cell proliferation, which results in inefficient DNA repair (Levine, A. Cell 88 (1997) 323-331). The most common changes of p53 in human cancer are point missense mutations, which are found in cancers of the colon, stomach, breast, lung, brain and esophagus (Greenblatt, M. et al., Cancer Res. 54 (1994) 4855-4878). It is estimated that p53 mutations is the most frequent genetic event in human cancers and accounts for more than 50% of cases. There is a very strong correlation between the frequency of p53 antibodies and the frequency of p53 mutations arguing that p53 mutations are involved in the appearance of these antibodies (Soussi, T. Cancer Res. 60 (2000) 1777-1788). P53 antibodies are found in human cancer patients with a specificity of 96%, but the sensitivity of such detection is only 30%.

Anti-p53 preferably is measured using two immuno-dominant peptides of the wild type p53 protein (Lubin et. al., Cancer Res. 53 (1993) 5872-5876).

As mentioned above, polypeptides, like seprase, are preferably measured by use of one or more specific binding agent(s). A specific binding agent is, e.g., a receptor for seprase, a lectin binding to seprase or an antibody to seprase. A specific binding agent has at least an affinity of 10⁷ l/mol for its corresponding target molecule. The specific binding agent preferably has an affinity of 10⁸ l/mol or also preferred of 10⁹ l/mol for its target molecule. As the skilled artisan will appreciate the term specific is used to indicate that other biomolecules present in the sample do not significantly bind to the binding agent specific for seprase. Preferably, the level of binding to a biomolecule other than the target molecule results in a binding affinity which is at most only 10% or less, only 5% or less only 2% or less or only 1% or less of the affinity to the target molecule, respectively. A preferred specific binding agent will fulfill both the above minimum criteria for affinity as well as for specificity.

A specific binding agent preferably is an antibody reactive with seprase. The term antibody refers to a polyclonal antibody, a monoclonal antibody, antigen binding fragments of such antibodies, single chain antibodies as well as to genetic constructs comprising the binding domain of an antibody.

Any antibody fragment retaining the above criteria of a specific binding agent can be used. Antibodies are generated by state of the art procedures, e.g., as described in Tijssen (Tijssen, P., Practice and theory of enzyme immunoassays, 11, Elsevier Science Publishers B.V., Amsterdam, the whole book, especially pages 43-78). In addition, the skilled artisan is well aware of methods based on immunosorbents that can be used for the specific isolation of antibodies. By these means the quality of polyclonal antibodies and hence their performance in immunoassays can be enhanced. (Tijssen, P., *supra,* pages 108-115).

For the achievements as disclosed in the present invention monoclonal as well as polyclonal antibodies, e.g. as raised in rabbits may be used. However, clearly also polyclonal antibodies from different species, e.g., rats or guinea pigs can also be used. Since monoclonal antibodies can be produced in any amount required with constant properties, they represent ideal tools in development of an assay for clinical routine. The generation and the use of monoclonal antibodies to seprase in a method according to the present invention, respectively, represent yet other preferred embodiments. Preferred examples of antibodies suitable for the detection of seprase are disclosed in Piñeiro-Sánchez et al., *supra,* e.g. the antibodies D8, D28 and D43.

For measurement of the marker panel according to the present invention an appropriate amount or aliquot of the sample obtained from an individual is incubated with the specific binding agents for different markers of the marker panel under conditions appropriate for formation of a binding agent marker complex. Such conditions need not be specified, since the skilled artisan without any inventive effort can easily identify such appropriate incubation conditions. The amount of binding agent marker complex is measured and used in the assessment of cancer, preferably of CRC. As the skilled artisan will appreciate there are numerous methods to measure the amount of the specific binding agent marker complex all described in detail in relevant textbooks (cf., e.g., Tijssen P., *supra,* or Diamandis, E.P. and Christopoulos, T.K. (eds.), Immunoassay, Academic Press, Boston (1996)).

In case the marker of interest is an antibody or an auto-antibody various detection method with which the skilled artisan is fully familiar are at hand. Non-limiting and preferred methods used in the detection of antibodies or as the case may be of auto-antibodies are the indirect sandwich assay method or the double-antigen-sandwich (DAGS) method. In the indirect sandwich method the (auto-)antibody (first antibody) is bound to the (auto-)antigen and indirectly detected via a labeled antibody (secondary antibody) to this (auto-)antibody or first antibody. In a DAGS method the assay is set up to provide for two antigens each comprising an epitope for the (auto-)antibody wherein one of the two antigens is bound to a solid phase or capable of binding to a solid phase and wherein the second antigen is detectably labeled. In case the (auto-)antibody is present a bridge it will bridge the first and the second antigen and the complex formed can be detected/measured.

In a preferred embodiment the present invention relates to a method for assessing CRC, in vitro by biochemical markers, comprising measuring in a sample the concentration of seprase and using the concentration and/or activity determined in the assessment of CRC.

In a further preferred embodiment the present invention relates to a method for assessing CRC, in vitro by biochemical markers, comprising measuring in a sample the concentration of seprase and of at least anti-p53, osteopontin or Ferritin, combining the concentration(s) and/or activity(ies) determined and using the combined value in the assessment of CRC.

The inventors of the present invention have surprisingly been able to detect a decreased concentration and/or activity of the marker seprase in a significant percentage of samples derived from patients with colon cancer. Again surprisingly they have been able to demonstrate that the decreased concentration and/or activity of seprase in such sample obtained from an individual can be used in the assessment of CRC. Even more surprising they have been able to demonstrate that the decreased concentration and/or activity of seprase in such sample obtained from an individual can be combined with at least a measurement of anti-p53, Ferritin and/or osteopontin and used in the assessment of CRC.

The ideal scenario for diagnosis would be a situation wherein a single event or process would cause the respective disease as, e.g., in infectious diseases. In all other cases correct diagnosis can be very difficult, especially when the etiology of the disease is not fully understood as is the case for many cancer types, e.g. for CRC. As the skilled artisan will appreciate, no biochemical marker is diagnostic with 100% specificity and at the same time 100% sensitivity for a given multifactorial disease, for example for CRC. Rather, biochemical markers, e.g., CYFRA21-1 or CEA, or as shown here certain marker combinations comprising seprase can be used to assess with a certain likelihood or predictive value e.g., the presence, absence, or the severity of a disease. Therefore in routine clinical diagnosis, generally various clinical symptoms and biological markers are considered together in the diagnosis, treatment and management of the underlying disease.

Biochemical markers can either be determined individually or in a preferred embodiment of the invention they can be measured simultaneously using a chip or a bead based array technology. The concentrations of the biomarkers are then combined for interpretation.

In the assessment of CRC the marker seprase or a panel comprising seprase and at least one marker selected from the group consisting of anti-p53, Ferritin and osteopontin will be of advantage in one or more of the following aspects: screening; diagnostic aid; prognosis; monitoring of therapy such as chemotherapy, radiotherapy, and immunotherapy.

### Screening:

Screening is defined as the systematic application of a test to identify individuals e.g. at risk individuals, for indicators of a disease, e.g., the presence of cancer. Preferably the screening population is composed of individuals known to be at higher than average risk of cancer. For example, a screening population for CRC may be composed of individuals known to be at higher than average risk for CRC, like persons at an age of 45 and above, or 50 and above or with a family history of CRC.

In the preferred embodiment a body fluid such as whole blood, serum or plasma is used as a sample in the screening for colorectal cancer.

For many diseases, no single biochemical marker in the circulation will ever meet the sensitivity and specificity criteria required for screening purposes. This appears to be also true for cancer and in particular for colorectal cancer. It has to be expected that a marker panel comprising a plurality of markers will have to be used in cancer screening. The data established in the present invention indicate that the marker seprase, preferably together with a marker selected from the group consisting of anti-p53, Ferritin and osteopontin will form an integral part of a marker panel appropriate for CRC screening purposes. The present invention therefore relates to the use of seprase as one marker of a CRC marker panel, for example a marker panel comprising the marker seprase together with a marker selected from the group consisting of anti-p53, Ferritin and osteopontin for CRC screening purposes. With reference to the preferred embodiment of screening for CRC, the present invention also relates to the use of a marker panel comprising seprase and CYFRA21-1, or of a marker panel comprising seprase and CEA, or to a marker panel comprising seprase and two or more markers selected from the group consisting of anti-p53, Ferritin, osteopontin, CYFRA21-1 and CEA..

### Diagnostic aid:

Markers may either aid the differential diagnosis of benign vs. malignant disease in a particular organ, help to distinguish between different histological types of a tumor, or to establish baseline marker values before surgery.

Today, important methods used in the detection of cancer are radiology and/or computed tomography (CT) scans (virtual colonoscopy). Small regions of suspect tissue can be visualized by these methods. However, many of these regions - more than 90% with CT - represent benign tissues changes, and only a minority of nodules represents cancerous tissue. Use of a marker panel comprising seprase and a marker selected from the group consisting of anti-p53, Ferritin and osteopontin may aid in the differentiation of benign versus malign regions.

Since seprase as a single marker might be superior to other markers, e.g. in the case of CRC to other markers, like CEA or CYFRA21-1, it has to be expected that seprase alone or together with other preferred markers as described in the present invention will be used as a diagnostic aid, especially by establishing a baseline value before surgery. The present invention thus also relates to the use of seprase or of marker combinations comprising seprase for establishing a baseline value before surgery for cancer.

### Prognosis:

Prognostic indicators can be defined as clinical, pathological, or biochemical features of cancer patients and their tumors that predict with certain likelihood the disease outcome. Their main use is to help to rationally plan patient management, i.e. to avoid undertreatment of aggressive disease and overtreatment of indolent disease, respectively.

As a marker panel according to the present invention significantly contributes to the differentiation of CRC patients, from controls, it has to be expected that it will aid in assessing the prognosis of patients suffering from CRC. The level of preoperative seprase will most likely be combined with one or more other marker for CRC and/or the TNM staging system. In one preferred embodiment a marker panel according to the present invention is used in the prognosis of patients with CRC.

### Monitoring of Chemotherapy:

Several reports have described the use of CEA in monitoring the treatment of patients with LC (e.g., Fukasawa T. et al., Cancer & Chemotherapy (1986) 13:1862-1867) or colorectal cancer, respectively. Most of these studies were retrospective, non-randomized and contained small numbers of patients. As in the case of the studies with CYFRA21-1 the CEA studies suggested: a) that patients with a decrease in CEA levels while receiving chemotherapy generally had a better outcome than those patients whose CEA levels failed to decrease and (b) for almost all patients, increases in CEA levels were associated with disease progression.

It is expected that a CRC marker panel according to present invention, i.e., a marker panel comprising seprase will be at least as good a marker for monitoring of chemotherapy in CRC as CEA. The present invention therefore also relates to a marker panel comprising seprase and at least one marker selected from the group consisting of anti-p53, Ferritin and/or osteopontin in the monitoring of cancer patients, preferably of CRC patients, under chemotherapy. In the monitoring of therapy in one preferred embodiment the measurements for seprase and for at least one marker selected from the group consisting of anti-p53, Ferritin and/or osteopontin and of optionally CYFRA 21-1 and/or CEA will be combined and used in the monitoring of CRC therapy.

### Follow-up:

A significant portion of CRC patients who undergo surgical resection aimed at complete removal of cancerous tissue later develop recurrent or metastatic disease. Most of these relapses occur within the first 2-3 years after surgery. Since recurrent/metastatic disease is invariably fatal if detected too late, considerable research has focused on detection of cancer relapse at an early and thus potentially treatable stage.

Consequently, many cancer patients, e.g. CRC patients undergo a postoperative surveillance program which frequently includes regular monitoring with CEA. Thus, the follow-up of patients with CRC after surgery is one of the most important fields of use for an appropriate biochemical marker. Due to the high sensitivity of seprase in the CRC patients investigated it is likely that seprase alone or in combination with one or more other marker will be of great help in the follow-up of CRC patients, especially in CRC patients after surgery. The use of a marker panel comprising seprase and one or more other marker of CRC in the follow-up of CRC patients represents a further preferred embodiment of the present invention. A further preferred embodiment relates to the use of a marker panel comprising seprase and at least one marker selected from the group consisting of anti-p53, Ferritin and/or osteopontin in the follow-up of CRC patients.

The present invention also relates to the use of seprase in the assessment of CRC.

As discussed above, certain preferred embodiments relate to the measurement values for seprase and for at least one marker selected from the group consisting of anti-p53, Ferritin and/or osteopontin to the combination of these measurement values and to the use of the combined value in the assessment of CRC.

As the skilled artisan will appreciate there are many ways to use the measurements of two or more markers in order to improve the diagnostic question under investigation. In a quite simple, but nonetheless often effective approach, a positive result is assumed if a sample is positive for at least one of the markers investigated. This may e.g. the case when diagnosing an infectious disease, like AIDS.

Frequently, however, the combination of markers is evaluated. Preferably the values measured for markers of a marker panel, e.g. for seprase and anti-p53, are mathematically combined and the combined value is correlated to the underlying diagnostic question. Marker values may be combined by any appropriate state of the art mathematical method. Well-known mathematical methods for correlating a marker combination to a disease employ methods like, discriminant analysis (DA) (i.e. linear-, quadratic-, regularized-DA), Kernel Methods (i.e. SVM), Nonparametric Methods (i.e. k-Nearest-Neighbor Classifiers), PLS (Partial Least Squares), Tree-Based Methods (i.e. Logic Regression, CART, Random Forest Methods, Boosting/Bagging Methods), Generalized Linear Models (i.e. Logistic Regression), Principal Components based Methods (i.e. SIMCA), Generalized Additive Models, Fuzzy Logic based Methods, Neural Networks and Genetic Algorithms based Methods. The skilled artisan will have no problem in selecting an appropriate method to evaluate a marker combination of the present invention. Preferably the method used in correlating the marker combination of the invention e.g. to the absence or presence of CRC is selected from DA (i.e. Linear-, Quadratic-, Regularized Discriminant Analysis), Kernel Methods (i.e. SVM), Nonparametric Methods (i.e. k-Nearest-Neighbor Classifiers), PLS (Partial Least Squares), Tree-Based Methods (i.e. Logic Regression, CART, Random Forest Methods, Boosting Methods), or Generalized Linear Models (i.e. Logistic Regression). Details relating to these statistical methods are found in the following references: Ruczinski, I., et al, J. of Computational and Graphical Statistics, 12 (2003) 475-511; Friedman, J. H., J. of the American Statistical Association 84 (1989) 165-175; Hastie, Trevor, Tibshirani, Robert, Friedman, Jerome, The Elements of Statistical Learning, Springer Series in Statistics, 2001; Breiman, L., Friedman, J. H., Olshen, R. A., Stone, C. J. (1984) Classification and regression trees, California: Wadsworth; Breiman, L., Random Forests, Machine Learning, 45 (2001) 5-32; Pepe, M. S., The Statistical Evaluation of Medical Tests for Classification and Prediction, Oxford Statistical Science Series, 28 (2003); and Duda, R. O., Hart, P. E., Stork, D. G., Pattern Classification, Wiley Interscience, 2nd Edition (2001).

It is a preferred embodiment of the invention to use an optimized multivariate cut-off for the underlying combination of biological markers and to discriminate state A from state B, e.g. diseased from no-diseased. In this type of analysis the markers are no longer independent but form a marker panel.

Accuracy of a diagnostic method is best described by its receiver-operating characteristics (ROC) (see especially Zweig, M. H., and Campbell, G., Clin. Chem. 39 (1993) 561-577). The ROC graph is a plot of all of the sensitivity/specificity pairs resulting from continuously varying the decision threshold over the entire range of data observed.

The clinical performance of a laboratory test depends on its diagnostic accuracy, or the ability to correctly classify subjects into clinically relevant subgroups. Diagnostic accuracy measures the test's ability to correctly distinguish two different conditions of the subjects investigated. Such conditions are for example health and disease or benign versus malignant disease.

In each case, the ROC plot depicts the overlap between the two distributions by plotting the sensitivity versus 1 - specificity for the complete range of decision thresholds. On the y-axis is sensitivity, or the true-positive fraction [defined as (number of true-positive test results)/(number of true-positive + number of false-negative test results)]. This has also been referred to as positivity in the presence of a disease or condition. It is calculated solely from the affected subgroup. On the x-axis is the false-positive fraction, or 1 - specificity [defined as (number of false-positive results)/(number of true-negative + number of false-positive results)]. It is an index of specificity and is calculated entirely from the unaffected subgroup. Because the true- and false-positive fractions are calculated entirely separately, by using the test results from two different subgroups, the ROC plot is independent of the prevalence of disease in the sample. Each point on the ROC plot represents a sensitivity/1-specificity pair corresponding to a particular decision threshold. A test with perfect discrimination (no overlap in the two distributions of results) has an ROC plot that passes through the upper left corner, where the true-positive fraction is 1.0, or 100% (perfect sensitivity), and the false-positive fraction is 0 (perfect specificity). The theoretical plot for a test with no discrimination (identical distributions of results for the two groups) is a 45° diagonal line from the lower left corner to the upper right corner. Most plots fall in between these two extremes. (If the ROC plot falls completely below the 45° diagonal, this is easily remedied by reversing the criterion for "positivity" from "greater than" to "less than" or vice versa.) Qualitatively, the closer the plot is to the upper left corner, the higher the overall accuracy of the test.

One preferred way to quantify the diagnostic accuracy of a laboratory test is to express its performance by a single number. Such an overall parameter e.g. is the so-called "total error" or alternatively the "area under the curve = AUC". The most common global measure is the area under the ROC plot. By convention, this area is always ≥ 0.5 (if it is not, one can reverse the decision rule to make it so). Values range between 1.0 (perfect separation of the test values of the two groups) and 0.5 (no apparent distributional difference between the two groups of test values). The area does not depend only on a particular portion of the plot such as the point closest to the diagonal or the sensitivity at 90% specificity, but on the entire plot. This is a quantitative, descriptive expression of how close the ROC plot is to the perfect one (area = 1.0).

In a preferred embodiment the present invention relates to a method for improving the diagnostic accuracy for CRC versus controls by measuring in a sample the concentration of at least seprase and a marker selected from the group consisting of anti-p53, Ferritin and/or osteopontin, respectively, mathematically combining the concentrations determined and correlating the combined value to the presence or absence of CRC, the improvement resulting in more patients being correctly classified as being at risk or suffering from CRC versus controls as compared to a classification based on any single marker investigated alone.

In a preferred method according to the present invention at least the concentration of the biomarkers seprase and a marker selected from the group consisting of anti-p53, Ferritin and/or osteopontin, respectively, is determined and the marker combination is used in the assessment of CRC.

In a preferred method according to the present invention at least the concentration of the biomarkers seprase and CYFRA21-1, respectively, is determined and the marker combination is used in the assessment of CRC.

In a further preferred method according to the present invention at least the concentration of the biomarkers seprase and CEA, respectively, is determined and the marker combination is used in the assessment of CRC.

In a further preferred method according to the present invention at least the measurement values for the biomarkers seprase, CEA, anti-p53 and Ferritin are combined for the assessment of CRC.

In a further preferred method according to the present invention at least the measurement values for the biomarkers seprase, CEA, anti-p53 and osteopontin are combined for the assessment of CRC.

In a preferred method according to the present invention at least the measurement results for seprase, CEA, anti-p53, CYFRA21-1 and osteopontin are determined, mathematically combined, and this marker combination is used in the assessment of CRC.

In a preferred method according to the present invention at least the measurement results for seprase, CEA, anti-p53, CYFRA21-1 and Ferritin are determined, mathematically combined, and this marker combination is used in the assessment of CRC.

In a further preferred method according to the present invention at least the measurement results for seprase, CEA, anti-p53, CYFRA21-1, osteopontin and Ferritin are determined, mathematically combined, and this marker combination is used in the assessment of CRC.

The following examples, sequence listing and the figures are provided to aid the understanding of the present invention, the true scope of which is set forth in the appended claims. It is understood that modifications can be made in the procedures set forth without departing from the spirit of the invention.

### Description of the Figures

- **Figure 1**: Flow chart of the patients selected for the study

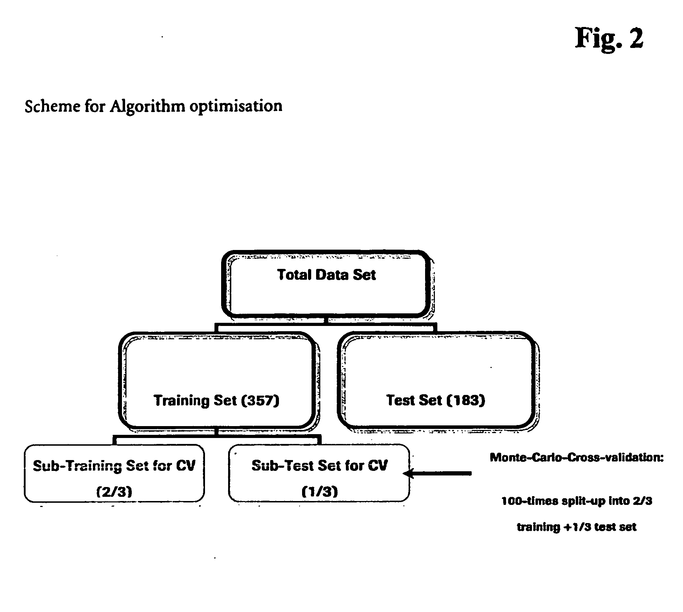

- **Figure 2**: Scheme for algorithm optimisation
- **Figure 3**: Selection of markers in Cross-validation. Figure 3 shows that in 100 statistical runs both seprase and CEA have been selected by the statistic procedure each and every time and that Ferritin and OPN had been selected 99 and 98 times respectively.

### Example 1

### Clinical Specimen

Serum samples were collected prospectively in two European multi-center studies. The first study recruited patients at gastroenterology units in an average-risk screening population, which underwent a preventive check by colonoscopy. From each participant a colonoscopy was performed at the participating centers with preparation and sedation used at each site. The serum samples were collected shortly before the colonoscopy was performed. The size and location of each lesion were recorded. Pathologist examined each surgical resection specimen on site to determine the diagnosis and the respective staging. Due to the low incidence of approx. 0.05% of CRC patients within the preventive screening population cancer patients were additionally recruited in a second prospective study at different surgery units. In this study the serum samples were also collected before surgery or colonoscopy. The diagnosis of colorectal cancer was confirmed by pathological staging of each patient by pathologists on site. The research protocols for both studies were reviewed and approved by the appropriate ethics committees and all participants gave written informed consent.

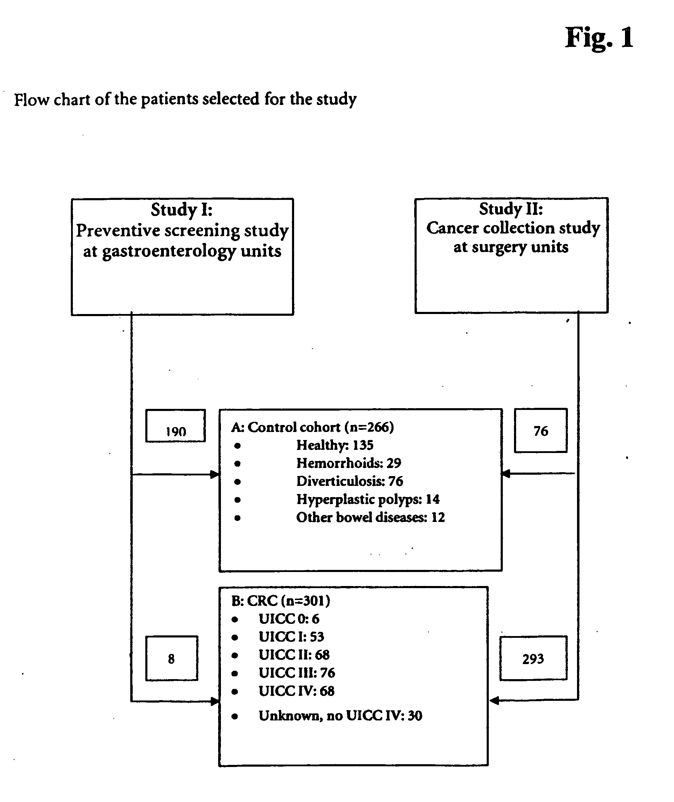

The clinical samples from both multi-center studies were compiled for the evaluation of the markers mentioned before (cf. Figure 1) resulting in a control cohort "A" and a CRC cohort "B", respectively, i.e.:
A: Control cohort with 266 patients. (No patients with adenoma or inflammatory bowel diseases had been included into the control cohort.)
B: CRC cohort with 301 colorectal cancer patients.

### Blood/serum collection:

Blood samples were drawn into a serum tube and allowed to clot for at least 1h and up to 2 h at room temperature. After centrifugation the supernatant (serum) was frozen at -25°C in 1 ml aliquots and transferred within two weeks to a -70°C freezer. Before measurement the serum samples were thawed and re-aliquoted into smaller volumes for convenience and refrozen. The samples were then thawed immediately before analysis. Therefore, every sample in the set was frozen and thawed only twice before analysis.

### Example 2

### Immunoassays for detection of tumor markers

### 2.1 Roche Elecsys^{®} Assays

Elecsys^{®} kits were acquired for following tumor markers: CEA, CA 15-3, CA 19-9, CA125, CA 72-4, Ferritin, ß-hCG, NSE, AFP and CYFRA21-1 (Cytokeratin-19). All assays were run according to the manufacturer's instructions on the Elecsys^{®} 2010 system (Roche Diagnostics GmbH, Mannheim, Germany). The concentrations measured by the instrument were used to calculate/generate the AUC data shown in Table 1.

### 2.2 Microtiter Plate Assays

For the markers seprase, osteopontin and anti-p53 micro titer assays were developed in-house.

### 2.2.1 Osteopontin (OPN)

For capture and detection of the antigen, two different antibodies were used. These antibodies were selected to have different non-overlapping epitopes. The epitopes of the two antibodies used are between amino acid 167 and the carboxy terminus of the osteopontin sequence (Kiefer, M.C. et al., Nucl. Acids Res. 17 (1989) 3306). One antibody had been biotinylated and was used as a capture antibody. The second antibody had been digoxigenylated. The digoxigenylated antibody was then detected by use of an appropriate anti-DIG secondary antibody.

### Assay Procedure:

Streptavidin-coated 96-well microtiter plates were incubated with 100 µl biotinylated anti-OPN polyclonal antibody for 60 min at 10 µg/mL in 10 mM phosphate buffered saline (PBS), pH 7.4, 1% BSA and 0.1% Tween 20. After incubation, plates were washed three times with phosphate buffered saline pH 7.4 (PBS), 0.1% Tween 20. Wells were then incubated for 2 h with either a serial dilution of the recombinant protein as standard antigen or with diluted plasma samples from patients. After binding of OPN, plates were washed three times with PBS, 0.1% Tween 20. For specific detection of bound OPN, wells were incubated with 100 µl of digoxigenylated anti-OPN polyclonal antibody for 60 min at 10 µg/ml in 10 mM phosphate, pH 7.4, 1% BSA, 0.9% NaCl and 0.1% Tween 20. Thereafter, plates were washed three times to remove unbound antibody. In a next step, wells were incubated with 20 mU/ml anti-digoxigenin-POD conjugates (Roche Diagnostics GmbH, Mannheim, Germany, Catalogue No. 1633716) for 60 min in 10 mM phosphate, pH 7.4, 1% BSA, 0,9% NaCl and 0.1% Tween 20. Plates were subsequently washed three times with the same buffer. For detection of antigen-antibody complexes, wells were incubated with 100 µl ABTS solution (Roche Diagnostics GmbH, Mannheim, Germany, Catalog No. 11685767) and OD was measured after 30-60 min at 405 nm with an ELISA reader.

### 2.2.2 anti-p53

The measurement of anti-p53 autoantibodies was carried out in duplicates in an indirect test format on streptavidin-coated microtiter plates. Two immunoreactive, biotinylated peptides
Bi-p53(11-35): Biotin-EPPLSQETFSDLWKLLPENNVLSPL (SEQ ID NO: 2) and
Bi-p53 (41-60): Biotin-DDLMLSPDDIEQWFTEDPGP (SEQ ID NO: 3), respectively,
were dissolved in PBS/ 0.05 % Tween 20 (50 ng/ml each) and immobilized on the streptavidin-coated microtiter plate (100 µl/well, 60 min, 30° C, shaking). After washing with PBS / 0.05 % Tween 20, diluted autoantibody-containing serum samples were incubated with the solid phase (Dilution: 1 : 50 with PBS / 0.05 % Tween 20; Volume: 100 µl/well, 120 min, 30° C, shaking). After washing with PBS / 0.0 5 % Tween 20, bound IgG was labeled with an anti-human-IgG-POD-Conjugate in an appropriate dilution. (Dilution in PBS/0.05 % Tween 20; 150 µl/well; incubation was performed for 60 min at 30° C under shaking). After washing, bound conjugate was detected using tetramethylbenzidine/hydrogenperoxide (TMB) as substrates (200 µl/well; 15 min; 30° C; shaking). The enzymatic reaction was stopped by the addition of 50 µl/well of 1 M sulfuric acid. The color was quantified photometrically at 450 nm using 620 nm as reference wave length. OD450 nm-values were converted in U/ml by a standard curve using a highly positive cancer serum. 1000 U/ml of the diluted cancer serum yielded about 3.0 OD450 nm under the above described assay conditions.

### 2.2.3 Seprase

As seprase-specific binding molecules two different rat monoclonal anti-seprase antibodies (clones D8 and D28) were used (Pineiro-Sanchez et al., M.L. et al., J. Biol. Chem. 12 (1997) 7595-7601).

### Biotinylation of monoclonal rat IgG:

Monoclonal rat IgG (clone D28) was brought to 10 mg/ml in 10 mM NaH₂PO₄/NaOH, pH 7.5, 30 mM NaCl. Per ml IgG solution 50 µl Biotin-N-hydroxysuccinimide (3.6 mg/ml in DMSO) were added. After 30 min at room temperature, the sample was chromatographed on Superdex 200 (10 mM NaH₂PO₄/NaOH, pH 7.5, 30 mM NaCl). The fractions containing biotinylated IgG were collected.

### Digoxygenylation of monoclonal rat IgG:

Monoclonal rat IgG (clone D8) was brought to 10 mg/ml in 10 mM NaH₂PO₄/NaOH, 30 mM NaCl, pH 7.5. Per ml IgG solution 50 µl of digoxigenin-3-O-methylcarbonyl-ε-aminocaproic acid-N-hydroxysuccinimide ester (Roche Diagnostics, Mannheim, Germany, Cat. No. 1 333 054) (3.8 mg/ml in DMSO) were added. After 30 min at room temperature, the sample was chromatographed on Superdex^{®} 200 (10 mM NaH₂PO₄/NaOH, pH 7.5, 30 mM NaCl). The fractions containing digoxigenylated IgG were collected.

ELISA for the measurement of seprase in human serum and plasma samples:
For detection of seprase in human serum or plasma, a sandwich ELISA was developed. Aliquots of the anti-seprase monoclonal antibodies D28 conjugated with biotin and D8 conjugated with digoxigenin (see above) were used for capturing and detection of the antigen.

Samples (75 µl) were mixed with 150 µl of antibody reagent containing 0.375 µg of each, D28-biotin and D8-digoxigenin antibodies in incubation buffer (comprising amongst other additives 40 mM phosphate, 200 mM sodium tartrate, 10 mM EDTA, 0.1% Tween 20, and adjusted to pH 7.4). After incubation for two hours, 2x100 µl of the mixture was transferred into separate wells of a streptavidin-coated microtiter plate and incubated for another hour. Plates were washed three times with washing buffer (10 mM Tris, 150 mM NaCl, 0.05% Tween 20). In a next step, wells were incubated with 30 mU/ml anti-digoxigenin-HRP conjugate (Roche Diagnostics GmbH, Mannheim, Germany, Catalogue # 1633716) in Universal Conjugate Buffer (Roche Diagnostics GmbH, Mannheim, Germany, Catalogue # 11684825) for 60 min and washed as before. Wells were then incubated for 1 hour with 100 µl of TMB substrate solution (Roche Diagnostics GmbH, Mannheim, Germany, Catalogue # 12034425). Adding of 2N sulfuric acid (50 µl) stopped the colour development and switched the blue colour into yellow. OD was measured at 450 nm with an ELISA reader (see above).

All incubations were at room temperature. Samples of human serum or plasma were pre-diluted with incubation buffer to a final concentration of 0.15 %. For calibration, a serum pool was used as a standard. It was diluted with incubation buffer to a final concentration of 0.09 / 0.18 / 0.27 / 0.36 / 0.45 % to make calibrators with arbitrarily given values of 0.03 / 0.06 / 0.09 / 0.12 / 0.15 Units/ml, respectively.

The equation of the calibration curve was calculated by linear regression analysis and used for converting the absorbance reading of a well into the corresponding concentration value. The result was multiplied by the pre-dilution factor to get the arbitrary seprase concentration of the respective sample itself.

### Example 3

### Univariate results of all markers

All serum samples were randomized and all markers were measured in our laboratory. For each microtiter plate assay the markers were assessed independently in different sample aliquots in duplicate and the mean concentration was used for further analysis. On the other hand all Elecsys^{®} parameters were measured from one single aliquot in single determination.

The cut-off for each assay was determined in the control collective A by cakulation of the 95% quantile resulting in a 95% specificity. The diagnostic potential of the biomarkers was evaluated either by calculating the receiver operator characteristic curves (ROC) or the clinical sensitivity at the preset specificity of 95% (Table 1).

**Table 1:**

| **ROC analysis and sensitivities of all single markers (301 CRC, 266 controls, at 95% specificity)** | | | | |
|---|---|---|---|---|
| **Marker** | **Assay** | **AUC (%)** | **Sensitivity all UICC stages (%)** | **Sensitivity UICC stages I-III** (%) |
| CEA | Elecsys | 77 | 43,9 | 30,9 |
| CA 15-3 | Elecsys | 53 | 4,0 | 3,4 |
| CA 125 | Elecsys | 56 | 17,6 | 10,3 |
| CA 19-9 | Elecsys | 64 | 22,6 | 15,0 |
| CA 72-4 | Elecsys | 66 | 20,6 | 12,9 |
| CYFRA21-1 | Elecsys | 78 | 35,5 | 22,3 |
| Ferritin | Elecsys | 65 | 23,9 | 27,0 |
| NSE | Elecsys | 57 | 14,0 | 6,4 |
| AFP | Elecsys | 56 | 6,6 | 5,6 |
| ß-hCG | Elecsys | 51 | 3,7 | 3,4 |
| OPN | Roche-MTP | 77 | 30,2 | 23,2 |
| Anti-p53 | Roche-MTP | 57 | 20,0 | 18,8 |
| Seprase | Roche-MTP | 79 | 42,4 | 45,4 |
| S100 A12 | Roche-MTP | 69 | 20,3 | 12,9 |
| HGF | MTP | 70 | 30,4 | 24,7 |
| TIMP-1 | MTP | 66 | 26,8 | 15,6 |
| IL-6 | MTP | 74 | 30,6 | 23,6 |
| IL-8 | MTP | 76 | 27,9 | 15,4 |

Some markers showed AUC values above 70%, i.e., CEA, CYFRA21-1, osteopontin, seprase and the inflammation markers IL-6 and IL-8. It is important to note, that Ferritin and Seprase both were down regulated in CRC, whereas all other markers in general are increased in CRC patients.

In order to simulate the screening situation the sensitivities were also calculated for all cancer stages without UICC stage IV. This calculation clearly indicates that the marker candidates Ferritin, anti-p53 and seprase are not influenced by elimination of UICC stage IV. Looking to the stage dependency of these 3 markers, no significant differences were found. Seprase and anti-p53, respectively, revealed the highest sensitivities in UICC stage I. Other markers are strongly influenced by tumor stage and showed the highest sensitivities for patients in stage IV, like CEA (see Table 2).

**Table 2:**

| **Sensitivities of individual markers for patients in different stages of CRC at 95% specificity** | | | | |
|---|---|---|---|---|
| Marker | Sensitivity (%) UICC I | Sensitivity UICC II | Sensitivity (%) UICC III | Sensitivity (%) UICC IV |
| CEA | 13,2 | 36,8 | 34,2 | 88,2 |
| CA 19-9 | 11,3 | 16,2 | 17,1 | 48,5 |
| CYFRA21-1 | 13,2 | 26,5 | 23,7 | 80,9 |
| Ferritin | 13,2 | 30,9 | 34,2 | 13,2 |
| OPN | 13,2 | 22,1 | 27,6 | 54,4 |
| Anti-p53 | 16,0 | 17,9 | 15,8 | 24,2 |
| Seprase | 36,0 | 49,3 | 51,3 | 31,8 |
| S100 A12 | 9,4 | 13,2 | 13,2 | 45,6 |
| HGF | 18,9 | 23,5 | 25,3 | 50,0 |
| TIMP-1 | 13,2 | 16,2 | 17,3 | 64,7 |
| IL-6 | 13,2 | 30,9 | 19,7 | 54,4 |
| IL-8 | 7,8 | 19,7 | 17,3 | 71,2 |

### Example 4

### Marker combinations / statistical analysis and results

Penalized Logistic Regression (PLR) was used as a mathematical model for marker combinations as implemented in the R-toolbox "penalized" (http://cran.r-project.org/). The total sample collective was randomly divided in a training set (2/3 of samples) and a test set (1/3 of samples). For the allocation of the patients to the two sample sets the variables like age, sex, collection site and distribution of tumor stages were considered. The algorithm optimisation (namely the selection of the penalization type and its penalization parameter) was carried out by 100 runs in a Monte-Carlo cross-validation design within the training set.

Within each Monte-Carlo cross-validation run two-thirds of all CRC and control cases of the training set were randomly selected as sub-training set. PLR was applied to the sub-training set to generate a diagnostic rule. A threshold on the estimated posterior case-probabilities was determined on the controls of the sub-training set to achieve an apparent specificity of 95% or 98% for the multivariate diagnostic rule. This rule was then applied to the remaining third of the data (sub-test set) to estimate sensitivity and specificity at the given threshold. This procedure was repeated 100 times.

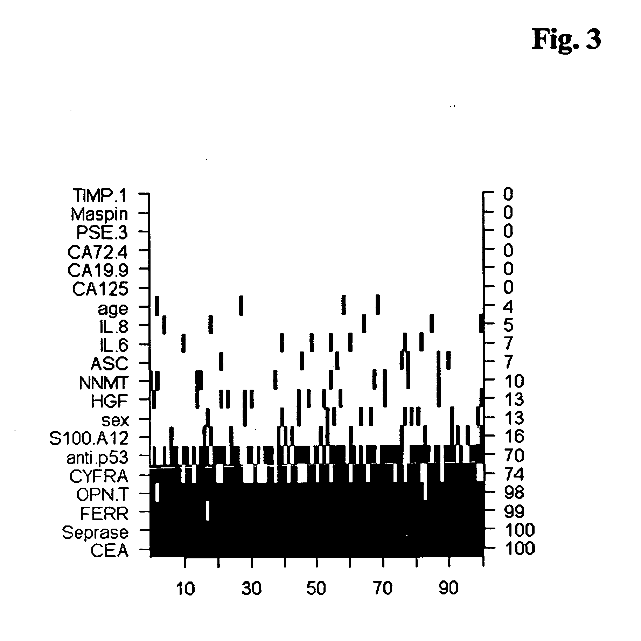

A close analysis of the individual runs from cross validation revealed that in 100 statistical runs both seprase and CEA have been selected by the statistic procedure each and every time while Ferritin and OPN had been selected 99 and 98 times respectively (see Figure 3). Cyfra 21-1 and anti-p53 were selected 74 and 70 times respectively while all the other markers tested were only selected less than 20 times or never selected.

All multivariate results of the CRC and control collective in the training set are therefore reported as median sensitivities from cross validation. The final diagnostic rule and its thresholds are then learnt with the optimised algorithm using all training data and are applied to the test set, which is used for validation of the generated diagnostic rule.

**Table 3:**

| **Results of best marker combination consisting of seprase, CEA, Ferritin, OPN, anti-p53 and CYFRA21-1** | | | | | |
|---|---|---|---|---|---|
| | **Training set*** | | | **Test set** | |
| 95% preset specificity | Sensitivity (%) sub-training | Sensitivity (%) sub-test | Specificity (%) sub-test | Sens. (%) | Spec. (%) |
| CRC cases 0-IV | 75 | 71 | 95 | 71 | 96 |
| CRC cases 0-III | 68 | 63 | 94 | 69 | 96 |

| 98% preset specificity | | | | | |
|---|---|---|---|---|---|
| CRC cases 0-IV | 65 | 62 | 98 | 60 | 97 |
| CRC cases 0-III | 59 | 54 | 98 | 53 | 98 |

| | | | | | |
|---|---|---|---|---|---|
| * Median of sub-training sets or sub-test-sets from 100 Monte-Carlo cross-validation runs | | | | | |

The results in Table 3 clearly show, that by combination of 6 markers the sensitivity can be significantly improved compared to the best single marker seprase without any loss of specificity. These results in serum samples are comparable or better than the results obtained with an immunological FOBT assay (Morikawa T. et. al., Gastroenterology 2005; 129:422-428).

In order to estimate the importance of each marker candidate in the final diagnostic rule each theoretically possible combination of these markers was assessed. For this purpose we used the samples of the training set and calculated the sensitivity of each marker combination in basically the same manner as the final rule, yet feeding the algorithm only with the data of the considered subset of markers. The sensitivity of colorectal carcinoma (only UICC stages I to III, without stage IV) was calculated for a preset specificity of 95% and 98% on the training data. Absolute values of these so-called apparent sensitivities are thus subject to overfit, the difference to the apparent sensitivity of the actual "final rule" though gives interpretable insight into the importance of individual markers, as it appropriately takes into account the correlation structure of the markers.

**Table 4:**

| **Results of different marker combinations on the training set** | | | | | | | |
|---|---|---|---|---|---|---|---|
| **Sepr.** | **CEA** | **Ferriti n** | **anti-p53** | **OPN** | **CYFRA 21-1** | **Sens. (%) at 95% spec.** | **Sens. (%) at 98% spec.** |
| X | | | | | | 51,7 | 32,4 |
| X | X | | | | | 60,7 | 40,7 |
| X | | X | | | | 55,2 | 45,5 |
| X | | | X | | | 60,0 | 37,2 |
| X | | | | X | | 51,7 | 37,2 |
| X | | | | | X | 45,5 | 43,4 |
| X | X | X | X | X | X | 69,0 | 61,4 |
| X | X | X | X | X | | 67,6 | 61,4 |
| X | X | X | X | | X | 67,6 | 51,7 |
| X | X | X | | X | X | 67,6 | 56,6 |
| X | X | | X | X | X | 66,9 | 53,1 |
| X | | X | X | X | X | 58,6 | 51,0 |
| | X | X | X | X | X | 51,7 | 44,8 |

The best results can be obtained by the combination of the six markers mentioned in Table 4. By leaving one marker out of the combination of six markers the sensitivity is slightly decreasing, with the exception of CYFRA21-1 at a preset specificity of 98%. On the other hand leaving out the most dominant marker, seprase, even the combination of all the other five markers only leads to a sensitivity comparable to the one achieved with seprase alone. The data in Table 4 also show that the best combination of 2 markers is dependent on the specificity level required. At 95% specificity the best two marker combinations are seprase + CEA and seprase + anti-p53, whereas at 98% specificity the combinations seprase + Ferritin and seprase + CYFRA21-1 are the most important ones. It is also obvious from the data presented in Table 4 that at the high specificity limit of 98% more markers are necessary to obtain the best results with regard to a high sensitivity. After leaving out one marker out of the six marker panel, at a preset specificity of 98% the decrease of sensitivity is higher as compared to the corresponding decrease at a preset specificity limit of 95 %.

## Claims

1. A method for assessing colorectal cancer (CRC) in vitro comprising measuring in a whole blood, serum or plasma sample the concentration and/or activity of
a) a seprase polypeptide and/or fragments thereof,
b) anti-p53, and/or osteopontin, and/or ferritin,
c) optionally one or more other marker of CRC, and
d) using the combined measurement result of step (a) and (b) and optionally of step (c) in the assessment of CRC, wherein a reduced concentration for seprase, a reduced concentration for ferritin, an increased concentration for anti-p53, and an increased concentration for osteopontin is indicative for CRC.

2. The method of claim 1, wherein at least the measurement results for seprase and anti-p53 are combined for assessment of CRC.

3. The method of claim 1, wherein at least the measurement results for seprase and osteopontin are combined for assessment of CRC.

4. The method of claim 1, wherein at least the measurement results for seprase and ferritin are combined for assessment of CRC.

5. The method of claim 1, wherein the optionally one or more other marker is selected from the group consisting of Carcinoembryonic antigen (CEA) and CYFRA21-1.

6. The method according to any of claims 1 to 3 and 5, wherein at least the measurement results for seprase, CEA, osteopontin and Ferritin are combined.

7. The method according to any of claims 1 to 6, wherein at least the measurement results for seprase, CEA, anti-p53, CYFRA21-1, CEA and Ferritin are combined.

8. The method according to any one of claims 1-7, wherein the concentration of a seprase polypeptide and/or fragments thereof and/or of complexes comprising seprase and/or a fragment thereof is measured.

9. Use in-vitro of a marker panel comprising at least the measurement results for seprase and anti-p53 for assessment of CRC.

10. Use in-vitro of a marker panel comprising at least the measurement results for seprase and osteopontin for assessment of CRC.

11. Use in-vitro of a marker panel comprising at least the measurement results for seprase and Ferritin for assessment of CRC.

12. Use in-vitro of a marker panel comprising at least the measurement results for seprase, CEA, anti-p53 and Ferritin for assessment of CRC.

13. Use in-vitro of a marker panel comprising at least the measurement results for seprase, CEA, anti-p53, CYFRA21-1, CEA and Ferritin for assessment of CRC.

14. A kit comprising antibodies for measuring the markers of claims 1-8 or for measuring the marker panels according to the uses of claims 9 to 13.

## Patentansprüche

1. Verfahren zum Bestimmen von Kolorektalkrebs (CRC) in vitro, bei dem man in einer Vollblut-, Serum- oder Plasmaprobe die Konzentration und/oder Aktivität der Folgenden in einer Vollblut-, Serum- oder Plasmaprobe misst von:
a) einem Seprasepolypeptid und/oder Fragmenten davon,
b) anti-p53 und/oder Osteopontin und/oder Ferritin,
c) gegebenenfalls einem oder mehreren anderen CRC-Markern, und
d) Verwendung des kombinierten Messergebnisses von Schritt (a) und (b) und gegebenenfalls von Schritt (c) für die Bestimmung von CRC, wobei eine verringerte Seprasekonzentration, eine verringerte Ferritinkonzentration, eine erhöhte anti-p53-Konzentration und eine erhöhte Osteopontinkonzentration CRC indizieren.

2. Verfahren nach Anspruch 1, wobei zumindest die Messwertergebnisse für Seprase und anti-p53 für die Bestimmung von CRC kombiniert werden.

3. Verfahren nach Anspruch 1, wobei zumindest die Messwertergebnisse für Seprase und Osteopontin für die Bestimmung von CRC kombiniert werden.

4. Verfahren nach Anspruch 1, wobei zumindest die Messwertergebnisse für Seprase und Ferritin für die Bestimmung von CRC kombiniert werden.

5. Verfahren nach Anspruch 1, wobei der gegebenenfalls ein oder mehrere andere Marker aus der Gruppe bestehend aus Carcinoembryonic-Antigen (CEA) und CYFRA21-1 ausgewählt ist.

6. Verfahren nach einem der Ansprüche 1 bis 3 und 5, wobei zumindest die Messergebnisse für Seprase, CEA, Osteopontin und Ferritin kombiniert werden.

7. Verfahren nach einem der Ansprüche 1 bis 6, wobei zumindest die Messergebnisse für Seprase, CEA, anti-p53, CYFRA21-1, CEA und Ferritin kombiniert werden.

8. Verfahren nach einem der Ansprüche 1-7, wobei die Konzentration eines Seprasepolypeptids und/oder von Fragmenten davon und/oder von Komplexen umfassend Seprase und/oder ein Fragment davon gemessen wird.

9. In-vitro-Verwendung eines Markerpanels, umfassend zumindest die Messergebnisse für Seprase und anti-p53 für die Bestimmung von CRC.

10. In-vitro-Verwendung eines Markerpanels, umfassend zumindest die Messergebnisse für Seprase und Osteopontin für die Bestimmung von CRC.

11. In-vitro-Verwendung eines Markerpanels, umfassend zumindest die Messergebnisse für Seprase und Ferritin für die Bestimmung von CRC.

12. In-vitro-Verwendung eines Markerpanels, umfassend zumindest die Messergebnisse für Seprase und CEA, anti-p53 und Ferritin für die Bestimmung von CRC.

13. In-vitro-Verwendung eines Markerpanels, umfassend zumindest die Messergebnisse für Seprase, CEA, anti-p53, CYFRA21-1, CEA und Ferritin für die Bestimmung von CRC.

14. Kit, umfassend Antikörper für die Messung der Marker nach den Ansprüchen 1-8 oder für die Messung der Markerpanels nach den Verwendungen der Ansprüche 9 bis 13.

## Revendications

1. Procédé pour évaluer un cancer colorectal (CRC) in vitro comprenant la mesure dans un échantillon de sang entier, de sérum ou de plasma, la concentration et/ou l'activité
a) d'un polypeptide de séprase et/ou d'un de ses fragments ;
b) de l'anticorps anti-p53 et/ou de l'ostéopontine et/ou de la ferritine ;
c) de manière facultative, d'un ou de plusieurs autres marqueurs du CRC ; et
d) l'utilisation des résultats de mesure combinés des étapes (a) et (b) et de manière facultative de l'étape (c) dans l'évaluation du CRC, dans lequel une réduction de la concentration pour la séprase, une réduction de la concentration pour la ferritine, une augmentation de la concentration pour l'anticorps anti-p53 et une augmentation de la concentration pour l'ostéopontine sont symptomatiques d'un CRC.

2. Procédé selon la revendication 1, dans lequel au moins les résultats de mesure pour la séprase et pour l'anticorps anti-p53 sont combinés pour l'évaluation du CRC.

3. Procédé selon la revendication 1, dans lequel au moins les résultats de mesure pour la séprase et pour l'ostéopontine sont combinés pour l'évaluation du CRC.

4. Procédé selon la revendication 1, dans lequel au moins les résultats de mesure pour la séprase et pour la ferritine sont combinés pour l'évaluation du CRC.

5. Procédé selon la revendication 1, dans lequel lesdits un ou plusieurs autres marqueurs facultatifs sont choisis parmi le groupe constitué par l'antigène carcinoembryonnaire (CEA) et le CYFRA21-1.

6. Procédé selon l'une quelconque des revendications 1 à 3 et 5, dans lequel au moins les résultats de mesure pour la séprase, le CEA, l'ostéopontine et la ferritine sont combinés.

7. Procédé selon l'une quelconque des revendications 1 à 6, dans lequel au moins les résultats de mesure pour la séprase, le CEA, l'anticorps anti-p53, le CYFRA21-1, le CEA et la ferritine sont combinés.

8. Procédé selon l'une quelconque des revendications 1 à 7, dans lequel on mesure la concentration d'un polypeptide de séprase et/ou d'un de ses fragments et/ou de complexes comprenant la séprase et/ou un de ses fragments.

9. Utilisation in vitro d'un panel de marqueurs comprenant au moins les résultats de mesure pour la séprase et l'anticorps anti-p53 pour l'évaluation du CRC.

10. Utilisation in vitro d'un panel de marqueurs comprenant au moins les résultats de mesure pour la séprase et l'ostéopontine pour l'évaluation du CRC.

11. Utilisation in vitro d'un panel de marqueurs comprenant au moins les résultats de mesure pour la séprase et la ferritine pour l'évaluation du CRC.

12. Utilisation in vitro d'un panel de marqueurs comprenant au moins les résultats de mesure pour la séprase, le CEA, l'anticorps anti-p53 et la ferritine pour l'évaluation du CRC.

13. Utilisation in vitro d'un panel de marqueurs comprenant au moins les résultats de mesure pour la séprase, le CEA, l'anticorps anti-p53, le CYFRA21-1, le CEA et la ferritine pour l'évaluation du CRC.

14. Trousse comprenant des anticorps pour la mesure des marqueurs selon les revendications 1 à 8 ou pour la mesure des panels de marqueurs selon les utilisations des revendications 9 à 13.
